# EUROPÄISCHE PATENTSCHRIFT

(11) **EP 4 093 018 B1**
(45) Veröffentlichungstag und Bekanntmachung des Hinweises auf die Patenterteilung: **31.12.2025**
(21) Anmeldenummer: 22174013.7
(22) Anmeldetag: 18.05.2022
(51) Int. Cl.: G06T 5/00, A61B 1/04, A61B 1/00, A61B 5/00, H04N 23/50, G01J 3/28, H04N 23/68, G01J 3/02, G02B 23/24

(54) **VERFAHREN ZUR MEDIZINISCHEN BILDGEBUNG UND MEDIZINISCHE BILDGEBUNGSVORRICHTUNG**
MEDICAL IMAGING METHOD AND MEDICAL IMAGING APPARATUS
PROCÉDÉ D'IMAGERIE MÉDICALE ET DISPOSITIF D'IMAGERIE MÉDICALE DESTINÉ

(30) Priorität: 19.05.2021 DE 102021112970
(43) Veröffentlichungstag der Anmeldung: 23.11.2022
(73) Patentinhaber: Karl Storz SE & Co. KG, 78532 Tuttlingen (DE)
(72) Erfinder: KÖHLER, Benedikt, 78532 Tuttlingen (DE); BUSCHLE, Lukas, 78532 Tuttlingen (DE)

(56) Entgegenhaltungen:
- EP-A1- 3 626 160
- EP-A2- 2 851 662
- US-A1- 2020 400 570

## Beschreibung

### Stand der Technik

Die Erfindung betrifft ein Verfahren zur medizinischen Bildgebung gemäß dem Oberbegriff des Anspruchs 1 sowie eine medizinische Bildgebungsvorrichtung zur Ausführung des Verfahrens gemäß Anspruch 12.

Aus der EP2851662A2 ist bereits ein Verfahren zur medizinischen Bildgebung bekannt, bei welchem in einem Aufnahmevorgang eine hyperspektrale Spektralabbildung eines Untersuchungsgebiets von einer endoskopischen und/oder exoskopischen hyperspektralen Spektralkamera durch zeilenweises zeitlich versetztes räumliches Scannen des Untersuchungsgebiets in mehreren Zeilenschritten aufgenommen wird. Dabei wird bei jedem Zeilenschritt eine räumliche Dimension und eine spektrale Dimension aufgenommen. Durch Aneinandersetzen der verschiedenen Zeilenschritte kann dann die Spektralabbildung erzeugt werden, welche eine Spektraldimension und zwei Raumdimensionen umfasst. Da die Zeilenschritte jedoch zeitlich nacheinander versetzt erfolgen, können durch Bewegungen der Spektralkamera und/oder eine Bewegung des Untersuchungsgebiets Bewegungsartefakte auftreten, welche eine Qualität der Bildgebung verringern oder diese sogar unbrauchbar machen können. Diese Bewegungsartefakte entstehen dadurch, dass durch die Bewegung Teile des Untersuchungsgebiets in verschiedenen Zeilenschritten mehrfach gescannt oder sogar übersprungen werden, wodurch es bei der Abbildung zu ungleichmäßigen Belichtungen und somit zu falschen Intensitäten der jeweiligen spektralen Information der Zeilenschritte kommt. Das gleiche Problem stellt sich auch für die Aufnahme von multispektralen Spektralabbildungen bzw. bei der Verwendung von multispektralen Spektralkameras, wobei dieses aufgrund der geringeren Anforderung an die spektrale Auflösung dort unter Umständen weniger prägnant sein kann.

Zudem offenbart Dokument US 2020/0400570 A1 ein Verfahren, umfassend eine Betätigung eines Emitters zum Aussenden von Impulsen elektromagnetischer Strahlung und ein Messen reflektierter elektromagnetischer Strahlung mit einem Pixelarray eines Bildsensors. Das Verfahren umfasst zudem das Erkennen von Bewegung zwischen zwei oder mehr aufeinanderfolgenden Belichtungsbildern, Kompensieren der Bewegung und Kombinieren der zwei oder mehr aufeinanderfolgenden Belichtungsbilder zur Erzeugung einer Abbildung.

Außerdem ist aus Dokument EP 3 626 160 A1 ein Abbildungselement zur Bereitstellung von Bilddaten, wie zum Beispiel für LSCI/LASCA-Messungen, bekannt, wobei das Abbildungselement eine Kamera, einen Laser, einen Bewegungsdetektor und eine Verarbeitungsvorrichtung umfasst. Die Verarbeitungsvorrichtung ist dazu eingerichtet, auf der Grundlage erfasster Bewegung ein Bild zu korrigieren.

Die Aufgabe der Erfindung besteht insbesondere darin, ein gattungsgemäßes Verfahren bzw. eine Vorrichtung mit verbesserten Eigenschaften hinsichtlich einer Qualität der Bildgebung bereitzustellen, bei welchem insbesondere Bewegungsartefakte berücksichtigt werden. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einem Verfahren zur medizinischen Bildgebung, bei welchem in einem Aufnahmevorgang wenigstens eine multi- und/oder hyperspektrale Spektralabbildung eines Untersuchungsgebiets von einer endoskopischen und/oder exoskopischen multi- und/oder hyperspektralen Spektralkamera aufgenommen wird.

Es wird vorgeschlagen, dass das Verfahren wenigstens einen Kollationsvorgang umfasst, in welchem Bewegungsartefakte in der Spektralabbildung, welche durch Bewegung der Spektralkamera und des Untersuchungsgebiets zueinander während des Aufnahmevorgangs auftreten, unter Berücksichtigung wenigstens einer Referenz der Spektralkamera und/oder des Untersuchungsgebiets zumindest teilweise, vorzugsweise zumindest zu einem Großteil und besonders bevorzugt vollständig, verringert und/oder vermieden werden, wobei anhand der Referenz eine Bewegung der Spektralkamera relativ zu dem Untersuchungsgebiet kenntlich gemacht und/oder erkannt wird.

Hierdurch kann vorteilhaft durch Berücksichtigung der Referenz eine Bewegung der Spektralkamera und des Untersuchungsgebiets zueinander erkannt werden, welche dann zu einer Warnung eines Benutzers bzgl. einer schlechteren Qualität der Spektralabbildung genutzt werden kann. Alternativ oder zusätzlich kann weiter vorteilhaft eine Anpassung und/oder Korrektur der Spektralabbildung auf Basis der Referenz vorgenommen werden. Somit können Bewegungsartefakte vermieden und/oder verringert werden. Insgesamt kann somit insbesondere eine Qualität der Spektralabbildung verbessert werden.

Unter einer "medizinischen Bildgebung" soll insbesondere eine Bildgebung verstanden werden, welche Rückschlüsse auf physiologische Eigenschaften eines Untersuchungsgebiets zulässt, wie beispielsweise Gewebeart und/oder Gewebeeigenschaften, wie beispielsweise einen Fettgehalt, einen Wassergehalt, eine Oxygenierung, ein Vorhandensein eines Farbstoffs oder dergleichen. Vorzugsweise nutzt die Bildgebung eine Spektralanalyse zur Bestimmung dieser physiologischen Eigenschaften. Bei dem Untersuchungsgebiet handelt es sich insbesondere um ein Gebiet, welches physiologische Bestandteile umfasst, wie beispielsweise Gewebe, Blut oder dergleichen. Das Untersuchungsgebiet liegt beispielsweise innerhalb einer natürlichen oder künstlich geschaffenen Kavität. Solche Kavitäten sind in etwa der Bauchraum, der Darm, die Blase, die Niere oder dergleichen. Allerdings könnte auch offenes Gewebe als Untersuchungsgebiet dienen. Unter einem "Aufnahmevorgang" soll insbesondere ein Vorgang zur Aufnahme verstanden werden, welcher wenigstens einen oder mehrere Verfahrensschritte umfasst. In dem Aufnahmevorgang der medizinischen Bildgebung wird eine Spektralabbildung oder Teilabschnitte der Spektralabbildung eines Untersuchungsgebiets mittels einer endoskopischen und/oder exoskopischen multi- und/oder hyperspektralen Spektralkamera aufgenommen. Unter einer "multi- und/oder hyperspektralen Spektralkamera" soll insbesondere eine Spektralkamera verstanden werden, welche zur Aufnahme von multi- und/oder hyperspektralen Abbildungen eingerichtet ist. Unter "eingerichtet" soll insbesondere speziell programmiert, vorgesehen, angepasst, ausgelegt und/oder ausgestattet verstanden werden. Darunter, dass ein Objekt zu einer bestimmten Funktion eingerichtet ist, soll insbesondere verstanden werden, dass das Objekt diese bestimmte Funktion in zumindest einem Anwendungs- und/oder Betriebszustand erfüllt und/oder ausführt. Zur Aufnahme der multi- und/oder hyperspektralen Spektralabbildung wird vorzugsweise die Technik des räumlichen Scannens bzw. Spatial-Scanning verwendet. Das Untersuchungsgebiet wird insbesondere zur Aufnahme der Spektralabbildung nacheinander in mehreren Aufnahmeschritten aus einzelnen Teilabschnitten erzeugt, und zwar insbesondere durch zeitlich versetztes zeilenweises Abscannen des Untersuchungsgebiets. Die Teilabschnitte können dann durch spektrale Auffächerung Aufschluss über eine Raumdimension und eine Spektraldimension geben. Aus den Teilabschnitten kann dann eine Spektralabbildung zusammengesetzt werden, wobei diese Teilabschnitte entlang einer weiteren Raumdimension zur Bildkonstruktion aneinandergesetzt werden. Die Spektralabbildung wird insbesondere von einem Datenwürfel gebildet, welcher die Raumdimensionen, die weitere Raumdimension und die Spektraldimension umfasst. Es sind aber auch weitere Techniken zur Aufnahme von Multi- und/oder Spektralabbildungen bekannt, wie beispielsweise Spectral-Scanning, Spatio-Spectral Scanning und/oder Non-Scanning bzw. Snapshot, bei welchen ebenfalls Bewegungsartefakte auftreten können, da diese zum Beispiel zeitlich versetzt unterschiedliche Spektralbänder aufnehmen und zu einem Spektralbild konstruieren. Auch sind je nach Anwendungsfall Mischformen dieser Methodiken bekannt. Es ist denkbar, dass die Spektralkamera zusätzlich eine stereoskopische Funktionsweise ermöglicht, mittels welcher zusätzliche dreidimensionale Rauminformationen aufgenommen werden können. In diesem Fall wird die Spektralabbildung von einem vierdimensionalen Tesserakt gebildet, welcher die Raumdimensionen, die weitere Raumdimension und die Spektraldimension sowie eine zusätzliche Raumdimension umfasst. Dies kann beispielsweise durch die Kombination zweier zueinander in ihrer Blickrichtung versetzt angeordneter Spektralkameras realisiert werden. Unter einer "multi- und/oder hyperspektralen Spektralabbildung" soll insbesondere eine Abbildung verstanden werden, welche spektrale Informationen von mehr als drei, vorzugsweise von mehr als zehn und besonders bevorzugt von mehr als hundert verschiedenen Farbkanälen umfasst und vorzugsweise zu einer Spektralanalyse basierend auf diesen Farbkanälen verwendet werden kann. Die Farbkanäle weisen insbesondere eine spektrale Halbwertsbreite von höchstens 80 nm, vorzugsweise höchstens 40 nm und besonders bevorzugt von höchstens 20 nm auf. Die Farbkanäle können einander wenigstens teilweise spektral überlappen, sind aber vorzugsweise voneinander zumindest im Wesentlichen distinkt ausgebildet. Im Bereich der Nutzung von mehr als drei und von weniger als zehn Farbkanälen spricht man im Allgemeinen von einer multispektralen Spektralabbildung, während man im Bereich der Nutzung von mehr als zehn Farbkanälen von einer hyperspektralen Abbildung spricht. Im Vergleich dazu umfasst beispielsweise eine üblicherweise mit einem R,G,B-Sensor aufgenommene Weißlichtabbildung lediglich spektrale Informationen dreier Farbkanäle, wie in etwa rot, grün und blau, die üblicherweise eine Halbwertsbreite von mehr als 80 nm aufweisen. Unter einer "endoskopischen Spektralkamera", welche zur Auffassung einer solchen multi- und/oder hyperspektralen Spektralabbildung dient, soll insbesondere eine Spektralkamera verstanden werden, welche mit einem Endoskop zusammenwirkt, gekoppelt bzw. koppelbar ist oder vorzugsweise mit diesem integral ausgebildet ist. Unter einer "exoskopischen Spektralkamera" soll insbesondere eine Spektralkamera verstanden werden, welche mit einem Exoskop zusammenwirkt, gekoppelt bzw. koppelbar ist oder vorzugsweise mit diesem integral ausgebildet ist. Unter einem "Exoskop" kann insbesondere auch ein chirurgisches Mikroskop verstanden werden. Bevorzugt soll unter einem Exoskop jedoch ein chirurgisches Mikroskop verstanden werden, welches frei von einem Okular ist und stattdessen vorzugsweise eine Kameraeinrichtung vorzugsweise mit wenigstens einer Weißlichtkamera umfasst.

Unter einem "Kollationsvorgang" soll insbesondere ein Vorgang zur Kollation verstanden werden, welcher wenigstens einen oder mehrere Verfahrensschritte umfasst. Der Kollationsvorgang kann zumindest teilweise gleichzeitig mit dem Aufnahmevorgang ablaufen. In dem Kollationsvorgang findet insbesondere wenigstens ein Abgleich mit wenigstens einer Referenz oder mehreren Referenzen statt. Die Referenz dient insbesondere dazu, eine Bewegung des Untersuchungsgebiets und/oder der Spektralkamera zu erkennen und vorzugsweise dazu eine Beziehung zwischen einer Bewegung des Untersuchungsgebiets und der Spektralkamera herzustellen. Insbesondere werden Bewegungsartefakte der Spektralabbildung unter Berücksichtigung der Referenz und insbesondere der basierend auf der Referenz erkannten Bewegung korrigiert. Alternativ kann ein Benutzer basierend auf der Referenz und insbesondere der basierend auf der Referenz erkannten Bewegung auf Bewegungsartefakte in der Multi- und/oder Hyperspektralabbildung, beispielsweise durch eine Markierung auf einem Bildschirm, hingewiesen werden oder sogar eine Spektralabbildung automatisch gelöscht werden.

Ferner wird vorgeschlagen, dass als wenigstens eine Referenz wenigstens eine Ziel-Position für die Spektralkamera relativ zu dem Untersuchungsgebiet sowie eine Ist-Position der Spektralkamera relativ zu dem Untersuchungsgebiet erfasst und ausgegeben wird, wobei durch kongruentes Anordnen der Ziel-Position und der Ist-Position Bewegungsartefakte vermieden werden. Es wird vorteilhaft einem Benutzer eine Zielvorrichtung bereitgestellt, welche es ihm erlaubt, Bewegungen des Untersuchungsgebiets und der Hyperspektralkamera während einer Aufnahme einer Multi- und/oder Hyperspektralabbildung zu vermeiden oder zu verringern. Die Ziel-Position ist insbesondere eine vorgesehene Position des Untersuchungsgebiets von welcher eine Spektralabbildung aufgenommen werden soll. Die Ist-Position entspricht insbesondere der Position der Spektralkamera im Vergleich zum Untersuchungsgebiet, insbesondere auch in Bezug auf einen Abstand zwischen der Spektralkamera und dem Untersuchungsgebiet. Die Ziel- Position und/oder die Ist-Position kann/können auf eine ebenfalls bereitgestellte Weißlichtabbildung und/oder das Untersuchungsgebiet selbst abgebildet werden. Durch kongruentes Anordnen der Ziel-Position und der Ist-Position können dann Abbildungsartefakte vermieden werden. Die Projektionen und die Weißlichtabbildung werden einem Benutzer auf einem Display oder auf dem Untersuchungsgebiet vorzugsweise in Echtzeit angezeigt. Die Ziel- und/oder Ist-Position kann/können beispielsweise durch ein Imagetracking anhand der Weißlichtabbildung, vorzugsweise in Verbindung mit einer stereoskopischen Triangulation, erfolgen und/oder durch eine Sensorik.

In einer bevorzugten Ausgestaltung wird vorgeschlagen, dass wenigstens eine Referenz von einer Sensorik bereitgestellt wird, welche eine Bewegung der Multi- und/oder Hyperspektralkamera und/oder des Untersuchungsgebiets erfasst. Hierdurch kann eine besonders verlässliche Erfassung der Bewegung und somit Vermeidung und/oder Bestimmung von Bewegungsartefakten erzielt werden. Bei der Sensorik kann es sich insbesondere um eine optische oder elektromagnetische Sensorik, wie beispielsweise ein optisches und/oder elektromagnetisches Trackingsystem, handeln. Ein solches Trackingsystem umfasst Marker, die an der Spetralkamera und/oder an dem Untersuchungsgebiet angeordnet sind. Alternativ oder zusätzlich kann die Sensorik Inertialsensoren, Beschleunigungssensoren, Hall-Sensoren oder dergleichen umfassen, welche an einem mit der Spektralkamera in Verbindung stehenden Endoskop/Exoskop, der Spektralkamera selbst und/oder an dem Untersuchungsgebiet angeordnet sind. Durch Überwachung der Sensorik kann auf eine Bewegung des Untersuchungsgebiets bzw. der Multi- und/oder Hyperspektralkamera geschlossen werden, welche zur Korrektur der Multi- und/oder Hyperspektralabbildung verwendet werden kann. Alternativ oder zusätzlich kann die Sensorik zur Erzeugung der Ist- und/oder Ziel-Position verwendet werden. Ferner kann die Sensorik dazu dienen, einen Abstand zwischen der Multi- und/oder Hyperspektralkamera und dem Untersuchungsgebiet zu bestimmen. Es kann vorteilhaft eine Bewegung der Multi- und/oder Hyperspektralkamera und des Untersuchungsgebiets genauer spezifiziert werden. Der Abstand kann über verschiedenste Methodiken ermittelt werden, jedoch vorzugsweise über eine Punktprojektion, Ultraschall, Time-of-flight, Stereorekonstruktion und/oder zeitlich versetzter perspektivischer Rekonstruktion.

Erfindungsgemäß wird vorgeschlagen, dass im Kollationsvorgang als wenigstens eine Referenz wenigstens eine auf die Multi- und/oder Hyperspektralabbildung kalibrierte Weißlichtabbildung des Untersuchungsgebiets bereitgestellt wird. Es kann vorteilhaft auf einfache Art und Weise eine Bewegung der Multi- und/oder Hyperspektralkamera und des Untersuchungsgebiets zueinander erkannt werden, basierend auf welcher die Hyperspektralabbildung nachbearbeitet werden kann, um Bewegungsartefakte auszugleichen. Die Weißlichtabbildung und die Spektralabbildung werden aufeinander abgebildet, um Abweichungen zwischen bestimmten Strukturen der Weißlichtabbildung und der Spektralabbildung zu erkennen. Dazu kann beispielsweise Feature-Tracking, in etwa nach Art des Motion-Capture, verwendet werden, bei welchem bestimmte Strukturen in der Weißlichtabbildung mit Strukturen in der Multi- und/oder Hyperspektralabbildung verglichen werden können. Ferner könnte auch für den Fall, dass sich aus dem Vergleich eine Bewegung dieser erkennen lässt, ein Benutzer darauf hingewiesen werden, dass Bewegungsartefakte erkannt wurden. Erfindungsgemäß werden die Bewegungsartefakte in der Spektralabbildung basierend auf der Bewegungserkennung mittels der Weißlichtabbildung korrigiert. Zur Bereitstellung der Weißlichtabbildung kann eine Bildkamera dienen, deren Position im Verhältnis zum Untersuchungsgebiet und/oder Spektralkamera bekannt ist. Dieses Verhältnis ist bevorzugt in einer Steuerelektronik wiederabrufbar hinterlegt. Besonders bevorzugt sind die Spektralkamera und eine Weißlichtkamera, welche zur Bereitstellung der Weißlichtabbildung dient, entlang einer gemeinsamen optischen Achse angeordnet. Ferner könnte die Weißlichtkamera an der Multi- und/oder Hyperspektralkamera befestigt oder in diese integriert werden, so dass deren Position zueinander stets fest gewählt ist, weshalb es keinem erneuten Abgleich dieser miteinander bedarf. Besonders bevorzugt sind die Spektralkamera und die Weißlichtkamera Teil einer gemeinsamen Kameraeinrichtung, welche mit dem Endoskop und/oder dem Exoskop in Verbindung steht, einer medizinischen Bildgebungsvorrichtung.

Ferner wird vorgeschlagen, dass im Kollationsvorgang wenigstens eine Weißlichtabbildung aus der Multi- und/oder Hyperspektralabbildung berechnet wird und mit der als Referenz dienenden Weißlichtabbildung verglichen wird. Hierdurch kann ein Vergleich der Spektralabbildung mit der Weißlichtabbildung vereinfacht werden, da bei Abweichung der aus der Multi- und/oder Hyperspektralabbildung berechneten Weißlichtabbildung von der als Referenz dienenden Weißlichtabbildung direkt auf eine fehlerhafte Bildgebung, aufgrund einer Bewegung geschlossen werden kann. Insbesondere wenn die Spektralabbildung über eine größere Anzahl von Farbkanälen verfügt und wenn die Quanteneffizienz dieser Farbkanäle und die eines R,G,B-Sensors, welcher zur Aufnahme der Weißlichtabbildung dient, bekannt sind, kann die aufgenommene Hyperspektralabbildung besonders vorteilhaft auf eine Weißlichtabbildung herunter gerechnet werden.

Weiterhin wird vorgeschlagen, dass während des Aufnahmevorgangs der Spektralabbildung die wenigstens eine als Referenz dienende Weißlichtabbildung des Untersuchungsgebiets aufgenommen wird. Hierdurch kann sichergestellt werden, dass eine erkannte Bewegung auch wirklich während einer Aufnahme der Multi- und/oder Hyperspektralabbildung stattgefunden hat und nicht davor oder danach. Vorzugsweise wird / werden die Weißlichtabbildungen wenigstens beim Start einer Aufnahme der Spektralabbildung und die weitere Weißlichtabbildung bei Beendigung der Aufnahme der Spektralabbildung aufgenommen. Aus der Differenz der beiden Weißlichtabbildungen kann dann, beispielsweise mittels eines Tracking-Algorithmus, das Stattfinden einer Bewegung zwischen Start und Beendigung der Aufnahme erkannt werden.

Eine Bildqualität kann insbesondere weiter verbessert werden, indem während einer Aufnahme der Spektralabbildung mehrere Weißlichtabbildungen aufgenommen werden. Dabei werden vorzugsweise die mehreren Weißlichtabbildungen mit einer Framerate von wenigstens 20 Hz erfasst. Dies erlaubt dann einen zuverlässigen Rückschluss, wann eine Bewegung, welche zur Verfälschung der Spektralabbildung führen kann, stattgefunden hat, so dass festgestellt werden kann, welche Teilabschnitte der Spektralabbildung noch unter Umständen verwendbar sind und welche nicht.

In einer bevorzugten Ausgestaltung wird vorgeschlagen, dass während des Aufnahmevorgangs nacheinander mehrere Aufnahmeschritte erfolgen, in welchen Teilabschnitte der Spektralabbildung aufgenommen werden, aus welchen diese zusammengesetzt werden, wobei pro Aufnahmeschritt wenigstens eine Weißlichtabbildung aufgenommen wird. Es kann vorteilhaft jeder Teilabschnitt auf seine Güte hinsichtlich eines möglichen Bewegungsartefakts hin untersucht werden. Bevorzugt wird dabei ein Teilabschnitt mit einer Aufnahmerate von wenigstens 100 Hz erfasst. Ganz besonders bevorzugt werden pro Aufnahmeschritt mehrere Weißlichtabbildungen aufgenommen.

In einem weiteren Aspekt der Erfindung wird eine medizinische Bildgebungsvorrichtung beansprucht, die wenigstens eine endoskopische und/oder exoskopische multi- und/oder hyperspektrale Spektralkamera umfasst, welche dazu eingerichtet ist, wenigstens eine multi- und/oder hyperspektrale Spektralabbildung eines Untersuchungsgebiets aufzunehmen, sowie wenigstens eine Steuerelektronik aufweist, welche zu einer Durchführung des obigen Verfahrens eingerichtet ist. Hierdurch kann vorteilhaft eine Vorrichtung bereitgestellt werden, welche eine Qualität der Bildgebung verbessert.

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer medizinischen Bildgebungsvorrichtung in einer perspektivischen Ansicht,
- Fig. 2: einen schematischen Aufbau einer Kameraeinrichtung der medizinischen Bildgebungsvorrichtung mit einer Spektralkamera und einer Weißlichtkamera,
- Fig. 3: einen schematischen Ablaufplan eines beispielhaften Verfahrens zum Betrieb der medizinischen Bildgebungsvorrichtung,
- Fig. 4: eine mittels der Kameraeinrichtung aufgenommene beispielhafte Weißlichtabbildung eines Untersuchungsgebiets mit voneinander abweichender Ist-Position und Zielposition als Referenz,
- Fig. 5: eine mittels der Kameraeinrichtung aufgenommene beispielhaftes Weißlichtabbildung des Untersuchungsgebiets mit kongruenter Ist-Position und Zielposition als Referenz,
- Fig. 6: eine mittels der Kameraeinrichtung aufgenommene Weißlichtabbildung des Untersuchungsgebiets,
- Fig. 7: eine mittels der Kameraeinrichtung aufgenommene Spektralabbildung, nach der Aufnahme der Weißlichtabbildung aus Fig. 6 mit Bewegungsartefakten,
- Fig. 8: eine mittels der Kameraeinrichtung aufgenommene Spektralabbildung der Fig. 7 mit Korrektur von Bewegungsartefakten.

### Beschreibung der Ausführungsbeispiele

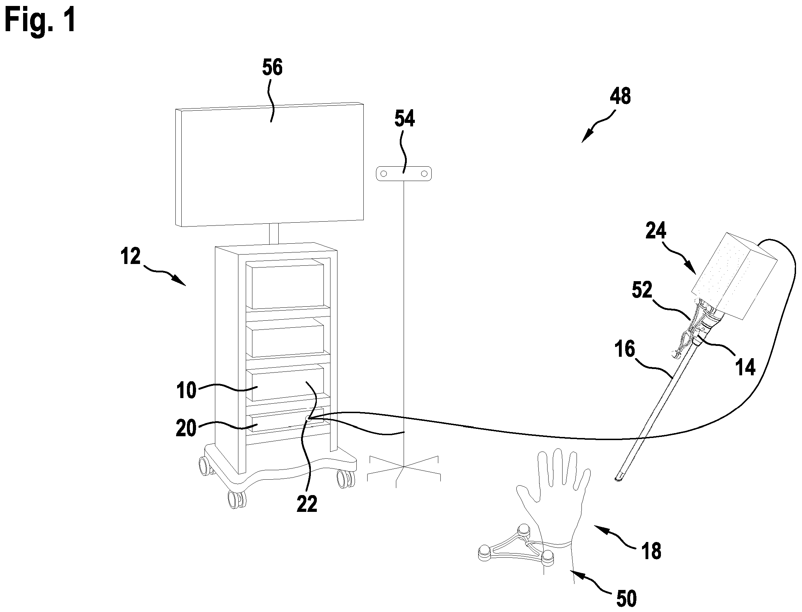

Fig. 1 zeigt eine schematische Darstellung einer medizinischen Bildgebungsvorrichtung in einer perspektivischen Ansicht. Im vorliegenden Fall bildet die medizinische Bildgebungsvorrichtung ein medizinisches Bildgebungssystem aus. Alternativ kann die medizinische Bildgebungsvorrichtung jedoch auch nur ein Teil des Systems sein.

Die medizinische Bildgebungsvorrichtung umfasst eine Steuerelektronik 10. Die Steuerelektronik 10 ist im vorliegenden Fall Teil eines separat ausgebildeten Steuergeräts 12 der medizinischen Bildgebungsvorrichtung. Die Steuerelektronik 10 ist zu einer Ansteuerung weiterer Komponenten der medizinischen Bildgebungsvorrichtung eingerichtet. Die Steuerelektronik 10 ist mit diesen weiteren Komponenten der medizinischen Bildgebungsvorrichtung verbunden. Die Steuerelektronik 10 ist zu einer Durchführung eines Verfahrens zur medizinischen Bildgebung eingerichtet. Die Steuerelektronik 10 weist wenigstens einen Prozessor (nicht dargestellt) auf. Ferner weist die Steuerelektronik 10 wenigstens einen Speicher (nicht dargestellt) auf. In dem Speicher ist ein Betriebsprogramm hinterlegt. Das Betriebsprogramm umfasst das Verfahren zur medizinischen Bildgebung. Das Betriebsprogramm ist von dem Prozessor ausführbar.

Die medizinische Bildgebungsvorrichtung umfasst ein Endoskop 14. Das Endoskop 14 ist im vorliegenden Fall als ein starres Endoskop ausgebildet. Bei dem Endoskop 14 könnte es sich aber auch um ein flexibles Endoskop handeln. Alternativ oder zusätzlich zu dem Endoskop könnte die medizinische Bildgebungsvorrichtung ein Exoskop umfassen.

Das Endoskop 14 weist einen Schaft 16 auf. Der Schaft 16 ist als ein längliches Bauteil ausgebildet. Im vorliegenden Fall ist der Schaft 16 starr ausgebildet. Der Schaft 16 ist dazu eingerichtet, bei einer Bildgebung mittels der medizinischen Bildgebungsvorrichtung in eine natürliche und/oder künstlichen geschaffene Kavität eingeführt zu werden.

Der Schaft 16 ist zu einer Übertragung von Licht in Richtung eines Untersuchungsgebiets 18 eingerichtet. Der besseren Veranschaulichung halber ist im vorliegenden Fall das Untersuchungsgebiet 18 in Form einer Hand dargestellt. Alternativ kann es sich bei dem Untersuchungsgebiet auch um eine natürliche und/oder künstliche Kavität oder eine andere physiologische Komponente handeln. Dazu weist der Schaft 16 einen Beleuchtungslichtleiter (nicht dargestellt) auf. Bei dem Beleuchtungslichtleiter kann es sich um einen Lichtwellenleiter, einen Linsenrelais oder dergleichen und insbesondere aber auch eine Kombination dieser handeln.

Ferner ist der Schaft 16 zu einer Übertragung von dem zum Untersuchungsgebiet 18 reflektierten, transmittierten und/oder abgegebenen, wie beispielsweise mittels Fluoreszenz oder Phosphoreszenz, Licht eingerichtet. Dazu weist der Schaft 16 wenigstens einen Untersuchungsgebietlichtleiter (nicht dargestellt) auf. Bei dem Untersuchungsgebietlichtleiter kann es sich um einen Lichtwellenleiter, einen Linsenrelais oder dergleichen und insbesondere aber auch eine Kombination dieser handeln. Ferner kann der Schaft 16 weitere Komponenten umfassen, wie beispielsweise einen Arbeitskanal, einen Fluidkanal oder dergleichen.

Die medizinische Bildgebungsvorrichtung weist wenigstens eine Beleuchtungseinrichtung 20 auf. Zu einer Ansteuerung der Beleuchtungseinrichtung 20 ist diese mit der Steuerelektronik 10 verbunden. Die Beleuchtungseinrichtung 20 ist Teil des Steuergeräts 12. Die Beleuchtungseinrichtung 20 weist wenigstens eine Lichtquelle 22 auf. Die Lichtquelle 22 ist dazu eingerichtet, Licht mit für eine gewünschte multi- und/oder hyperspektrale Bildgebung geeigneter spektraler Verteilung abzustrahlen. Im vorliegenden Fall handelt es sich bei der Lichtquelle 22 um eine hyperspektrale Lichtquelle. Diese emittiert ein breitbandiges Spektrum, welches sich über den gesamten sichtbaren Spektralbereich erstreckt. Ferner erstreckt sich diese zumindest teilweise über den NIR-Spektralbereich. Zur Erzeugung des abzustrahlenden Lichts weist die Lichtquelle 22 wenigstens ein Leuchtelement (nicht dargestellt) auf. Bei dem Leuchtelement kann es sich um eine LED, eine OLED, eine Weißlichtlampe, einen Laser, oder dergleichen handeln. Im vorliegenden Fall handelt es sich bei dem Leuchtelement um ein Breitbandfluoreszenz-LED. Denkbar kann die Lichtquelle 22 wenigstens zwei oder eine Mehrzahl von Leuchtelementen aufweisen, welche sich insbesondere in ihren spektralen Abstrahlcharakteristik voneinander unterscheiden können. Es ist denkbar, dass die mehreren Leuchtelemente abwechselnd getaktet oder zumindest teilweise gleichzeitig aktiviert werden können, um eine gewünschte spektrale Abstrahlcharakteristik des Lichts zur Beleuchtung des Untersuchungsgebiets bereitzustellen und/oder anzupassen. Im vorliegenden Fall einer hyperspektralen Bildgebung ist/wäre eine spektral breitbandige Lichtquelle sinnvoll, wie beispielsweise eine Weißlichtlichtquelle. Für den Fall der Nutzung zur multispektralen Bildgebung kann die Lichtquelle vorzugsweise verschiedene schmalbandige LEDs umfassen.

Die Bildgebungsvorrichtung weist eine Kameraeinrichtung 24 auf. Die Kameraeinrichtung 24 ist zu einer Ansteuerung mit der Steuerelektronik 10 verbunden. Die Kameraeinrichtung 24 ist mit dem Endoskop 14 koppelbar. In einem Betriebszustand ist die Kameraeinrichtung 24 mit dem Endoskop 14 gekoppelt.

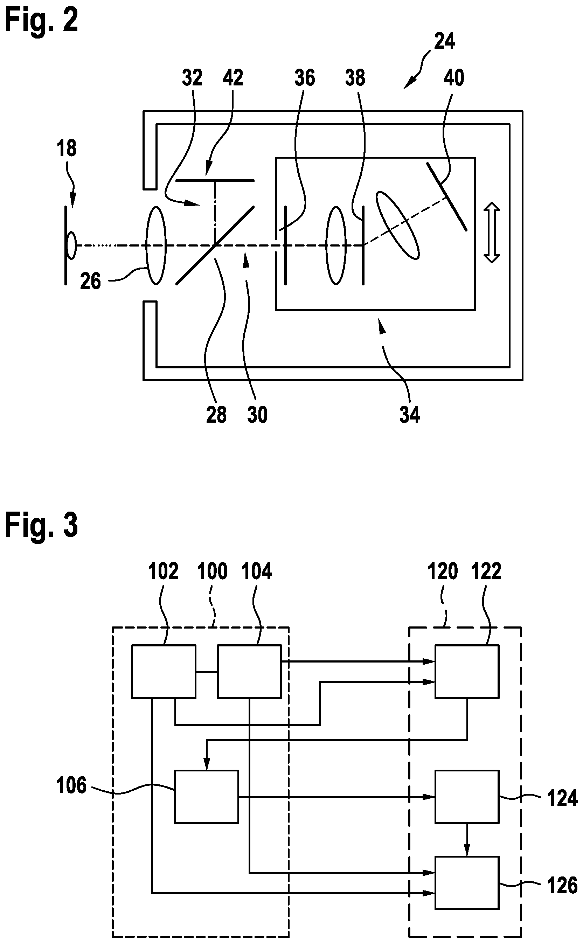

In Fig. 2 ist ein schematischer Aufbau der Kameraeinrichtung 24 der medizinischen Bildgebungsvorrichtung gezeigt. Die Kameraeinrichtung 24 weist ein Eingangsobjektiv 26 auf. Ferner weist die Kameraeinrichtung 24 einen Strahlteiler 28 auf, welcher lichtstromaufwärts hinter dem Eingangsobjektiv 26 angeordnet ist. Der Strahlteiler 28 teilt durch das Eingangsobjektiv 26 einfallendes Licht, welches vom Untersuchungsgebiet 18 ausgesandt wurde, in einen ersten Strahlengang 30 und einen zweiten Strahlengang 32 auf.

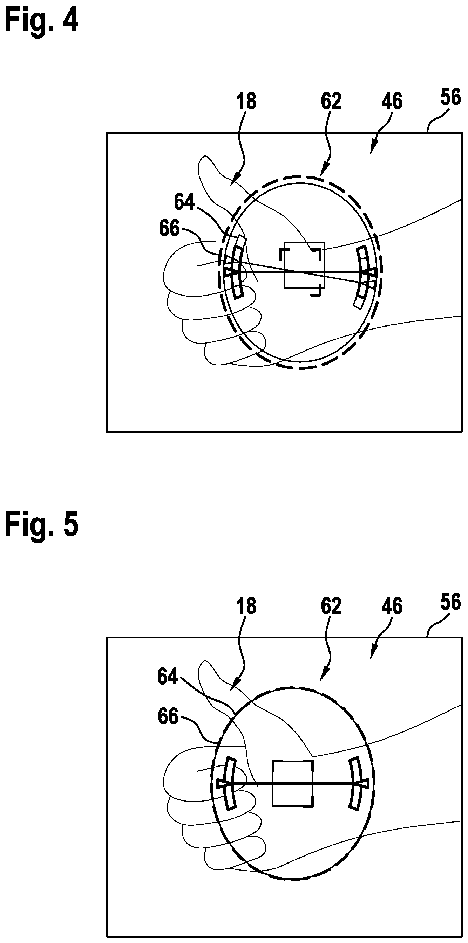

Die Kameraeinrichtung 24 weist im vorliegenden Fall eine Spektralkamera 34 auf. Im vorliegenden Fall ist die Spektralkamera 34 als eine Hyperspektralkamera ausgebildet. Alternativ kann diese auch als eine Multispektralkamera ausgebildet sein. Die Spektralkamera 34 ist lichtstromaufwärts hinter dem Strahlteiler 28 angeordnet. Die Spektralkamera 34 steht im ersten Strahlengang 30. Im vorliegenden Fall ist die Spektralkamera 34 als eine Spectral-Scanning Hyperspektralkamera ausgebildet. Diese bildet mittels eines beweglichen Spalts 36 und eines dispersiven Elements 38 das Untersuchungsgebiet 18 abschnittsweise auf einen Bildsensor 40 ab. Das dispersive Element 38 spaltet das Licht in seine spektralen Anteile auf. Indem die Spektralkamera 34 bzw. der Spalt 36 relativ zum ersten Strahlengang 30 bewegt wird, kann aus Teilabschnitten eine Spektralabbildung zusammengesetzt werden. In anderen Worten scannt die Spektralkamera 34 das Untersuchungsgebiet 18 zeitversetzt Schritt für Schritt, also insbesondere Zeile für Zeile, ab und setzt daraus eine Spektralabbildung 44 (vgl. Fig. 4) zusammen.

Ferner weist die Kameraeinrichtung 24 wenigstens eine Weißlichtkamera 42 auf. Die Weißlichtkamera 42 ist lichtstromaufwärts hinter dem Strahlteiler 28 angeordnet. Die Weißlichtkamera 42 steht im zweiten Strahlengang 32. Die Weißlichtkamera 42 dient zur Erzeugung einer Weißlichtabbildung 46. Die Weißlichtkamera 42 und die Spektralkamera 34 sind in einem festen Verhältnis zueinander angeordnet, so dass bekannt ist, wie die von der Spektralkamera 34 aufgenommene Spektralabbildung 44 und die von der Weißlichtkamera 42 aufgenommene Weißabbildung 46 zueinander in Beziehung stehen. Auch sind diese somit aufeinander abbildbar.

Die medizinische Bildgebungsvorrichtung weist ferner wenigstens eine Sensorik 48 auf (vgl. Fig. 1). Die Sensorik 48 ist dazu eingerichtet, eine Bewegung der Kameraeinrichtung 24 bzw. des Endoskops 14, und damit der Spektralkamera 34 bzw. der 42, relativ zum Untersuchungsgebiet 18 zu erfassen. Zur Überwachung ist die Sensorik 48 mit der Steuerelektronik 10 verbunden. Im vorliegenden Fall ist die Sensorik 48 als ein optisches Trackingsystem ausgebildet. Alternativ könnte es sich jedoch um ein elektromagnetisches Trackingsystem handeln.

Die Sensorik 48 umfasst wenigstens einen Trackingmarker 50. Der Trackingmarker 50 ist am Untersuchungsgebiet 18 angeordnet. Ferner umfasst die Sensorik 48 einen weiteren Trackingmarker 52. Der weitere Trackingmarker 52 ist an der Kameraeinrichtung 24 angeordnet. Ferner umfasst die Sensorik 48 eine Trackingkamera 54. Die Trackingkamera 54 erfasst die Trackingmarker 50, 52, wodurch die Positionen bzw. Bewegungen des Untersuchungsgebiets 18 und der Kameraeinrichtung 24 ermittelt werden können. Beispielsweise für den Fall, dass Trackingmarker nicht an dem Untersuchungsgebiet und/oder der Kameraeinrichtung anordenbar sind, ist es denkbar, dass alternativ oder zusätzlich die Sensorik einen Beschleunigungssensor oder dergleichen umfassen kann, welcher an der Spektralkamera angeordnet ist, um deren Ausrichtung zu bestimmen. Ferner könnte die Sensorik alternativ oder zusätzlich einen Abstandsmesser aufweisen. Dieser könnte mittels Time-of-flight, Ultraschall und/oder mittel Stereorekonstruktion den Abstand zwischen Untersuchungsgebiet und Kameraeinrichtung bestimmen.

Die medizinische Bildgebungsvorrichtung umfasst wenigstens ein Anzeigemittel 56. Bei dem Anzeigemittel 56 handelt es sich im vorliegenden Fall um einen Bildschirm. Das Anzeigemittel 56 ist mit der Steuerelektronik 10 verbunden. Das Anzeigemittel 56 ist zu einer Ausgabe von Informationen, wie beispielsweise einer Weißlichtabbildung, einer Spektralabbildung, eines Overlays oder dergleichen eingerichtet.

In Fig. 3 ist ein schematischer Ablaufplan eines beispielhaften Verfahrens zum Betrieb der medizinischen Bildgebungsvorrichtung gezeigt. Das Verfahren ist Teil des auf der Steuerelektronik 10 hinterlegten Betriebsprogramms. Das Verfahren wird von der Steuerelektronik 10 ausgeführt.

Das Verfahren umfasst wenigstens einen Aufnahmevorgang 100. Ferner umfasst das Verfahren wenigstens einen Kollationsvorgang 120. Der Aufnahmevorgang 100 und der Kollationsvorgang 120 können zumindest zeitweise gleichzeitig ablaufen.

Der Aufnahmevorgang 100 umfasst einen Aufnahmeschritt 102. In dem Aufnahmeschritt 102 wird mittels der Sensorik 48 die Position der Kameraeinrichtung bestimmt. Ferner wird die Position des Untersuchungsgebiets 18 mittels der Sensorik 48 bestimmt. Diese dient im Kollationsschritt 120 als eine Referenz. Der Aufnahmeschritt 102 erfolgt kontinuierlich während des Aufnahmevorgangs 100.

Der Aufnahmevorgang 100 umfasst einen weiteren Aufnahmeschritt. In dem weiteren Aufnahmeschritt wird mit der Weißlichtkamera 42 der Kameraeinrichtung 24 wenigstens eine Weißlichtabbildung 46 aufgenommen. Im vorliegenden Fall werden mehrere Weißlichtabbildungen 46 aufgenommen. Eine solche Weißlichtabbildung 46 ist beispielhaft in Fig. 4 dargestellt. Dies erfolgt mit einer Bildfrequenz von wenigstens 20 Hz. Im vorliegenden Fall erfolgt dies sogar mit einer Bildfrequenz von 100 Hz. Die Steuerelektronik 10 fragt die Informationen der Sensorik 48 und die Weißlichtabbilder 46 zeitgleich ab, so dass eine der Weißlichtabbildungen 46 stets einer mit der Sensorik 48 bestimmten Position der Spektralkamera 34 und des Untersuchungsgebiets 18 zuordenbar ist. Die Weißlichtabbildungen dienen als eine Referenz im Kollationsvorgang 120. Der Aufnahmeschritt 104 wird kontinuierlich während des Aufnahmevorgangs 100 ausgeführt.

Der Kollationsvorgang 120 umfasst wenigstens einen Kollationsschritt 122. In dem Kollationsschritt 122 fragt die Steuerelektronik 10 als Referenz die von der Sensorik 48 erfasste Position der Kameraeinrichtung 24 relativ zum Untersuchungsgebiet 18 ab. Ferner fragt die Steuereinrichtung 10 als weitere Referenz die von der Kameraeinrichtung 24 aufgenommenen Weißlichtabbildungen 46 ab. Dabei ordnet die Steuerelektronik 10 jeder Weißlichtabbildung 46 eine Ist-Position 64 der Spektralkamera 34 relativ zum Untersuchungsgebiet 18 zu (vgl. Fig. 4 und Fig. 5). Somit wird eine Ist-Position der Kameraeinrichtung 24 relativ zu dem Untersuchungsgebiet 18 zu jeder Weißlichtabbildung bekannt.

In Fig. 4 und 5 ist jeweils eine Weißlichtabbildung 46 des Untersuchungsgebiets 18 zusammen mit einem Overlay 62 aus einer Ist-Position 64 und einer Ziel-Position 66 dargestellt. Im Kollationsschritt wird in Form eines Overlays 62 die Ist-Position 64 der Spektralkamera 34 zusammen mit einer Weißlichtabbildung 46 auf dem Anzeigemittel 56 angezeigt. Ferner wird ein Benutzer dazu aufgerufen in der Weißlichtabbildung 46, beispielsweise durch eine Gesten- oder Touchbedienung, eine Ziel-Position 66 im Untersuchungsgebiet 18 festzulegen. Die Ziel-Position 66 dient dann als Referenz im Overlay 62 und wird zusammen mit der Ist-Position 64 angezeigt. Die Ist-Position 64 ist durch die von der Sensorik 48 erfassten Daten bestimmt. Der Bediener kann durch Bewegen der Spektralkamera 34 die Ziel-Position 66 und Ist-Position 64 kongruent aufeinander ausrichten. Sind Ziel-Position 66 und Ist-Position 64 kongruent, kann eine Aufnahme einer Spektralabbildung 44 gestartet werden.

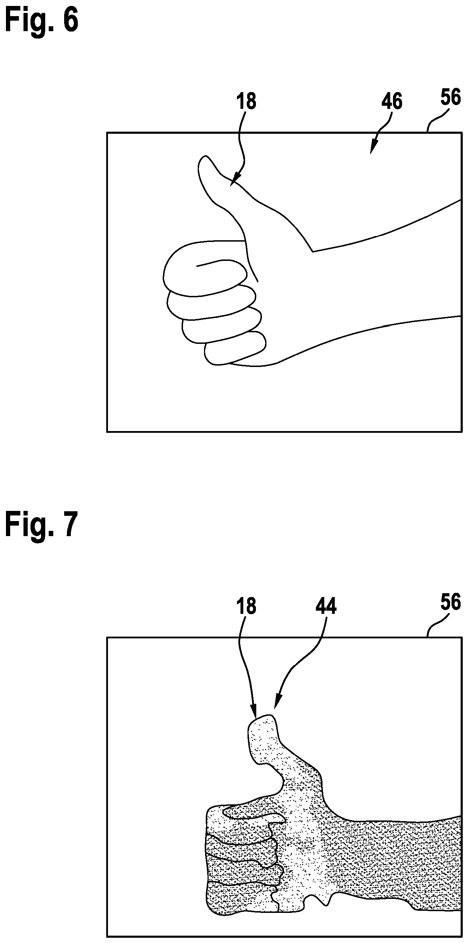

Der Aufnahmevorgang 100 umfasst einen weiteren Aufnahmeschritt 106. In dem Aufnahmeschritt 106 wird eine Spektralabbildung 44 aufgenommen. In. Fig. 6 und 7 ist beispielsweise eine Spektralabbildung 44 zusammen mit einer korrespondierenden Weißlichtabbildung 46 dargestellt. Die Aufnahme der Spektralabbildung 44 erfolgt dabei mittels der Spectral-Scanning Methode. Das Untersuchungsgebiet 18 wird in Teilschritten, welche einzelne Teilabschnitte der Spektralabbildung 44 umfasst, zeitlich versetzt Schritt für Schritt abgescannt. Die Teilabschnitte werden zu der Spektralabbildung 44 zusammengesetzt. Jeder Teilschritt erfolgt dabei mit einer Frequenz von 100 Hz. Da auch die Weißlichtabbildungen 46 mit dieser Frequenz aufgenommen werden, liegt zu jedem dieser Teilschritte eine Weißlichtabbildung 46 vor, welche als Referenz zur Korrektur der Spektralabbildung 44 herangezogen werden können.

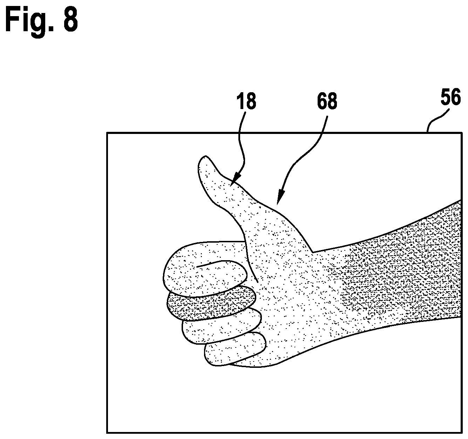

Der Kollationsvorgang 120 umfasst einen weiteren Kollationsschritt 124. In dem Kollationsschritt, wird aus der Spektralabbildung eine weitere Weißlichtabbildung berechnet. Diese kann als eine weitere Referenz genutzt werden. Die weitere Weißlichtabbildung wird dazu mit der zuvor aufgenommenen Weißlichtabbildung verglichen. Weichen diese voneinander ab, so ist während der Aufnahme eine Bewegung aufgetreten. Wenn diese gleich sind, trat keine Bewegung auf. Dem Benutzer wird ausgegeben, ob es zu einer Bewegung gekommen ist. Alternativ oder zusätzlich können die Daten der Sensorik während der Aufnahme als Referenz zur Feststellung einer solchen Bewegung herangezogen werden. Hat eine Bewegung und Bewegungsartefakte erzeugt, wie dies beispielsweise in Fig. 7 zu sehen ist, stattgefunden, wird dem Benutzer dies angezeigt. Daraufhin kann die Spektralabbildung verworfen werden. Alternativ kann in einem weiteren Kollationsschritt versucht werden, die Spektralabbildung basierend auf der Referenz der während der Aufnahme aufgenommenen Weißlichtabbildungen und/oder Daten der Sensorik zu korrigieren. Dies ist beispielsweise in den Fig. 8 gezeigt.

Der Kollationsvorgang 120 umfasst wenigstens einen weiteren Kollationsschritt 126. In dem Kollationsschritt 126 werden die Teilabschnitte der Spektralabbildung 46 mit jeweiligen zeitlich zugeordneten Weißlichtabbildungen 46 verglichen, da die Aufnahme der Weißlichtabbildung mit der gleichen Bildfrequenz erfolgt. Somit kann für jeden Teilabschnitt festgestellt werden, ob eine Bewegung stattgefunden hat und eine entsprechende Korrektur erfolgen. Auf Grundlage dieser wird dann aus der Spektralabbildung eine korrigierte Spektralabbildung erzeugt. In Fig. 8 ist die Weißlichtabbildung 46 zusammen mit der aus der Spektralabbildung 44 korrigierten Spektralabbildung 68 dargestellt.
- 10: Steuerelektronik
- 12: Steuergerät
- 14: Endoskop
- 16: Schaft
- 18: Untersuchungsgebiet
- 20: Beleuchtungseinrichtung
- 22: Lichtquelle
- 24: Kameraeinrichtung
- 26: Eingangsobjektiv
- 28: Strahlteiler
- 30: Erster Strahlengang
- 32: Zweiter Strahlengang
- 34: Spektralkamera
- 36: Spalt
- 38: Dispersives Element
- 40: Bildsensor
- 42: Weißlichtkamera
- 44: Spektralabbildung
- 46: Weißlichtabbildung
- 48: Sensorik
- 50: Trackingmarker
- 52: Weiterer Trackingmarker
- 54: Trackingkamera
- 56: Anzeigemittel
- 62: Overlay
- 64: Ist-Position
- 66: Ziel-Position
- 68: Korrigierte Spektralabbildung

- 100: Aufnahmevorgang
- 102: Aufnahmeschritt
- 104: Aufnahmeschritt
- 106: Aufnahmeschritt
- 120: Kollationsvorgang
- 122: Kollationsschritt
- 124: Kollationsschritt
- 126: Kollationsschritt

## Patentansprüche

1. Verfahren zur medizinischen Bildgebung, bei welchem in einem Aufnahmevorgang (100) wenigstens eine multi- und/oder hyperspektrale Spektralabbildung (44) eines Untersuchungsgebiets (18) von einer endoskopischen und/oder exoskopischen multi- und/oder hyperspektralen Spektralkamera (34) aufgenommen wird, wobei während des Aufnahmevorgangs (100) das Untersuchungsgebiet (18) nacheinander in mehreren Aufnahmeschritten aus einzelnen Teilabschnitten durch zeitlich versetztes zeilenweises Abscannen des Untersuchungsgebiets (18) erzeugt wird, und aus den Teilabschnitten die Spektralabbildung (44) zusammengesetzt wird, wobei die Teilabschnitte durch spektrale Auffächerung Aufschluss über eine Raumdimension und eine Spektraldimension geben, wobei das Verfahren wenigstens einen Kollationsvorgang (120) aufweist, in welchem Bewegungsartefakte in der Spektralabbildung (44), welche durch Bewegung der Spektralkamera (34) und des Untersuchungsgebiets (18) zueinander während des Aufnahmevorgangs (100) auftreten, unter Berücksichtigung wenigstens einer Referenz der Spektralkamera (34) und/oder des Untersuchungsgebiets (18) zumindest teilweise verringert und/oder vermieden werden, wobei anhand der Referenz eine Bewegung der Spektralkamera (34) relativ zu dem Untersuchungsgebiet (18) kenntlich gemacht und/oder erkannt wird, und wobei in dem Kollationsvorgang (120) wenigstens ein Abgleich mit der Referenz stattfindet, **dadurch gekennzeichnet, dass** im Kollationsvorgang (120) als wenigstens eine Referenz wenigstens eine auf die Spektralabbildung (44) kalibrierte Weißlichtabbildung (46) des Untersuchungsgebiets (18) bereitgestellt wird, wobei die Weißlichtabbildung (46) und die Spektralabbildung (44) aufeinander abgebildet werden, um Abweichungen zwischen bestimmten Strukturen der Weißlichtabbildung (46) und der Spektralabbildung (44) zu erkennen, und wobei die Bewegungsartefakte in der Spektralabbildung (44) basierend auf einer Bewegungserkennung mittels der Weißlichtabbildung (46) korrigiert werden.

2. Verfahren nach Anspruch 1, **dadurch gekennzeichnet, dass** als wenigstens eine Referenz wenigstens eine Ziel-Position (66) für die Spektralkamera (34) relativ zu dem Untersuchungsgebiet (18) sowie eine Ist-Position (64) der Spektralkamera (34) relativ zu dem Untersuchungsgebiet (18) erfasst und ausgegeben wird, wobei durch kongruentes Anordnen der Ziel-Position (66) und der Ist-Position (64) Bewegungsartefakte vermieden werden.

3. Verfahren nach einem der Ansprüche 1 oder 2, **dadurch gekennzeichnet, dass** im Kollationsvorgang (120) wenigstens eine Referenz von einer Sensorik (48) bereitgestellt wird, welche eine Bewegung der Spektralkamera (34) und/oder des Untersuchungsgebiets (18) erfasst.

4. Verfahren nach einem der vorhergehenden Ansprüche, **dadurch gekennzeichnet, dass** im Kollationsvorgang (120) wenigstens eine Weißlichtabbildung (46) aus der Spektralabbildung (44) berechnet wird und mit der als Referenz dienenden Weißlichtabbildung (46) verglichen wird.

5. Verfahren nach einem der vorhergehenden Ansprüche, **dadurch gekennzeichnet, dass** während des Aufnahmevorgangs (100) der Spektralabbildung (44) die wenigstens eine als Referenz dienende Weißlichtabbildung (46) des Untersuchungsgebiets (18) aufgenommen wird.

6. Verfahren nach Anspruch 5, **dadurch gekennzeichnet, dass** während des Aufnahmevorgangs (100) mehrere als Referenz dienende Weißlichtabbildungen (46) des Untersuchungsgebiets (18) aufgenommen werden.

7. Verfahren nach Anspruch 6, **dadurch gekennzeichnet, dass** die mehreren Weißlichtabbildungen (46) mit einer Bildfrequenz von wenigstens 20 Hz erfasst werden.

8. Verfahren nach Anspruch 6 oder 7, **dadurch gekennzeichnet, dass** während des Aufnahmevorgangs (100) nacheinander mehrere Aufnahmeschritte erfolgen, in welchen Teilabschnitte der Spektralabbildung (44) aufgenommen werden, aus welchen diese zusammengesetzt wird, wobei pro Aufnahmeschritt wenigstens eine Weißlichtabbildungen (46) aufgenommen wird.

9. Verfahren nach Anspruch 8, **dadurch gekennzeichnet, dass** während des Aufnahmevorgangs (100) die Aufnahmeschritte mit einer Aufnahmerate von wenigstens 100 Hz ablaufen.

10. Verfahren nach Anspruch 8 oder 9, **dadurch gekennzeichnet, dass** pro Aufnahmeschritt der Spektralabbildung (44) mehrere Weißlichtabbildungen (46) aufgenommen werden.

11. Medizinische Bildgebungsvorrichtung mit wenigstens einer endoskopischen und/oder exoskopischen multi- und/oder hyperspektralen Spektralkamera (34), welche dazu eingerichtet ist, wenigstens eine multi- und/oder hyperspektrale Spektralabbildung (44) eines Untersuchungsgebiets (18) aufzunehmen, und mit wenigstens einer Steuerelektronik (10), welche zu einer Durchführung eines Verfahrens nach einem der Ansprüche 1 bis 10 eingerichtet ist.

12. Medizinische Bildgebungsvorrichtung nach Anspruch 11, **dadurch gekennzeichnet, dass sie** ein Endoskop und/oder ein Exoskop **umfasst,** welches mit der multi- und/oder hyperspektralen Spektralkamera (34) verbindbar ist, verbunden ist und/oder mit dieser integral ausgebildet ist.

## Claims

1. Method for medical imaging, in which, in a capture process (100), at least one multi- and/or hyperspectral spectral image (44) of an examination region (18) is captured by an endoscopic and/or exoscopic multi- and/or hyperspectral spectral camera (34), the examination region (18) being generated successively during the capture process (100) in a plurality of capture steps from individual subsections by temporally offset line-by-line scanning of the examination region (18), and the spectral image (44) being composed of the subsections, the subsections providing information about a spatial dimension and a spectral dimension by spectral fanning-out, the method comprising at least one collation process (120) in which motion artifacts in the spectral image (44) caused by movement of the spectral camera (34) and the examination region (18) relative to one another during the capture process (100) are at least partially reduced and/or eliminated taking into account at least one reference of the spectral camera (34) and/or of the examination region (18), a movement of the spectral camera (34) relative to the examination region (18) being identified and/or recognized on the basis of the reference, and at least one comparison with the reference taking place in the collation process (120), **characterized in that,** in the collation process (120), at least one white-light image (46) of the examination region (18) calibrated to the spectral image (44) is provided as at least one reference, the white-light image (46) and the spectral image (44) being imaged onto one another in order to recognize discrepancies between certain structures in the white-light image (46) and the spectral image (44), and the motion artifacts in the spectral image (44) being corrected based on movement recognition by means of the white-light image (46).

2. Method according to claim 1, **characterized in that** at least one target position (66) for the spectral camera (34) relative to the examination region (18) and an actual position (64) of the spectral camera (34) relative to the examination region (18) are detected and output as at least one reference, motion artifacts being eliminated by arranging the target position (66) and the actual position (64) congruently.

3. Method according to either one of claims 1 and 2, **characterized in that,** in the collation process (120), at least one reference is provided by a sensor system (48) which detects a movement of the spectral camera (34) and/or of the examination region (18).

4. Method according to any one of the preceding claims, **characterized in that,** in the collation process (120), at least one white-light image (46) is computed from the spectral image (44) and is compared with the white-light image (46) serving as a reference.

5. Method according to any one of the preceding claims, **characterized in that,** during the capture process (100) for capturing the spectral image (44), the at least one white-light image (46) of the examination region (18) serving as a reference is captured.

6. Method according to claim 5, **characterized in that,** during the capture process (100), a plurality of white-light images (46) of the examination region (18) serving as reference are captured.

7. Method according to claim 6, **characterized in that** the plurality of white-light images (46) are detected at an image frequency of at least 20 Hz.

8. Method according to claim 6 or 7, **characterized in that,** during the capture process (100), a plurality of capture steps are carried out one after the other, in which subsections of the spectral image (44) are captured, of which subsections said spectral image is composed, at least one white-light images (46) being captured per capture step.

9. Method according to claim 8, **characterized in that,** during the capture process (100), the capture steps take place at a capture rate of at least 100 Hz.

10. Method according to claim 8 or 9, **characterized in that** a plurality of white-light images (46) are captured per capture step of capturing the spectral image (44).

11. Medical imaging device having at least one endoscopic and/or exoscopic multi- and/or hyperspectral spectral camera (34) which is configured to capture at least one multi- and/or hyperspectral spectral image (44) of an examination region (18), and having at least one control electronics unit (10) which is configured to carry out a method according to any one of claims 1 to 10.

12. Medical imaging device according to claim 11, **characterized in that it comprises** an endoscope and/or an exoscope, which is connectable to, connected to and/or integrally formed with the multi- and/or hyperspectral spectral camera (34).

## Revendications

1. Procédé pour l'imagerie médicale, dans lequel, au cours d'un processus de prise de vue (100), au moins une représentation spectrale (44) multi- et/ou hyperspectrale d'une zone d'examen (18) est capturée par une caméra spectrale (34) multi- et/ou hyperspectrale endoscopique et/ou exoscopique, dans lequel la zone d'examen (18) est générée pendant le processus de prise de vue (100), successivement en plusieurs étapes de prise de vue, à partir de sections partielles individuelles, par balayage ligne par ligne et décalé dans le temps de la zone d'examen (18) et la représentation spectrale (44) est assemblée à partir des sections partielles, dans lequel les sections partielles donnent des informations sur une dimension spatiale et une dimension spectrale par répartition spectrale, dans lequel le procédé présente au moins un processus de collationnement (120) dans lequel des artefacts de déplacement dans la représentation spectrale (44), lesquels apparaissent par un déplacement de la caméra spectrale (34) et de la zone d'examen (18) l'une par rapport à l'autre pendant le processus de prise de vue (100), sont au moins partiellement réduits et/ou évités en prenant en compte au moins une référence de la caméra spectrale (34) et/ou de la zone d'examen (18), dans lequel un déplacement de la caméra spectrale (34) par rapport à la zone d'examen (18) est reconnu et/ou rendu reconnaissable à l'aide de la référence, et dans lequel au moins un alignement avec la référence a lieu dans le processus de collationnement (120), **caractérisé en ce que,** dans le processus de collationnement (120), au moins une représentation de lumière blanche (46) de la zone d'examen (18) calibrée sur la représentation spectrale (44) est fournie en tant qu'au moins une référence, dans lequel la représentation de lumière blanche (46) et la représentation spectrale (44) sont représentées l'une sur l'autre afin de reconnaître des écarts entre certaines structures de la représentation de lumière blanche (46) et de la représentation spectrale (44), et dans lequel les artefacts de déplacement dans la représentation spectrale (44) sont corrigés sur la base d'une reconnaissance de déplacement au moyen de la représentation de lumière blanche (46).

2. Procédé selon la revendication 1, **caractérisé en ce qu'**au moins une position cible (66) pour la caméra spectrale (34) par rapport à la zone d'examen (18) ainsi qu'une position réelle (64) de la caméra spectrale (34) par rapport à la zone d'examen (18) sont détectées et émises en tant qu'au moins une référence, dans lequel des artefacts de déplacement sont évités par une disposition coïncidente de la position cible (66) et de la position réelle (64).

3. Procédé selon l'une des revendications 1 ou 2, **caractérisé en ce que,** dans le processus de collationnement (120), au moins une référence est fournie par un système à capteurs (48) qui détecte un déplacement de la caméra spectrale (34) et/ou de la zone d'examen (18).

4. Procédé selon l'une des revendications précédentes, **caractérisé en ce que,** dans le processus de collationnement (120), au moins une représentation de lumière blanche (46) est calculée à partir de la représentation spectrale (44) et est comparée à la représentation de lumière blanche (46) servant de référence.

5. Procédé selon l'une des revendications précédentes, **caractérisé en ce que,** pendant le processus de prise de vue (100) de la représentation spectrale (44), l'au moins une représentation de lumière blanche (46) servant de référence de la zone d'examen (18) est capturée.

6. Procédé selon la revendication 5, **caractérisé en ce que,** pendant le processus de prise de vue (100), plusieurs représentations de lumière blanche (46) servant de référence de la zone d'examen (18) sont capturées.

7. Procédé selon la revendication 6, **caractérisé en ce que** les représentations de lumière blanche (46) sont détectées à une fréquence d'images d'au moins 20 Hz.

8. Procédé selon la revendication 6 ou 7, **caractérisé en ce que,** pendant le processus de prise de vue (100), plusieurs étapes de prise de vue sont effectuées successivement, dans lesquelles des sections partielles de la représentation spectrale (44) sont capturées, à partir desquelles celle-ci est assemblée, dans lequel au moins une représentation de lumière blanche (46) est capturée par étape de prise de vue.

9. Procédé selon la revendication 8, **caractérisé en ce que,** pendant le processus de prise de vue (100), les étapes de prise de vue se déroulent à une fréquence de prise de vue d'au moins 100 Hz.

10. Procédé selon la revendication 8 ou 9, **caractérisé en ce que** plusieurs représentations de lumière blanche (46) sont capturées par étape de prise de vue de la représentation spectrale (44).

11. Dispositif d'imagerie médicale comportant au moins une caméra spectrale (34) multi- et/ou hyperspectrale endoscopique et/ou exoscopique configurée pour capturer au moins une représentation spectrale (44) multi- et/ou hyperspectrale d'une zone d'examen (18), et comportant au moins un équipement électronique de commande (10) configuré pour mettre en œuvre un procédé selon l'une des revendications 1 à 10.

12. Dispositif d'imagerie médicale selon la revendication 11, **caractérisé en ce qu'il comprend** un endoscope et/ou un exoscope qui sont connectés et/ou peuvent être connectés à la caméra spectrale (34) multi- et/ou hyperspectrale et/ou sont réalisés d'un seul tenant avec celle-ci.
